# EUROPÄISCHE PATENTSCHRIFT

(11) **EP 0 812 817 B1**
(45) Veröffentlichungstag und Bekanntmachung des Hinweises auf die Patenterteilung: **25.07.2001**
(21) Anmeldenummer: 97106416.7
(22) Anmeldetag: 18.04.1997
(51) Int. Cl.: C07C 67/00, C07C 69/34

(54) **Verfahren zur Herstellung von Diestern höherer alpha, omega-Dicarbonsäuren**
Process for preparing di-esters of higher alpha, omega-dicarboxylic acids
Procédé pour la préparation de hauts-esters d'acides alpha, omega-dicarboxyliques

(30) Priorität: 13.06.1996 DE 19623585
(43) Veröffentlichungstag der Anmeldung: 17.12.1997
(73) Patentinhaber: Degussa AG, 40474 Düsseldorf (DE)
(72) Erfinder: Köhler, Günther, Dr., 45770 Marl (DE); Metz, Josef, Dr., 45770 Marl (DE)

(56) Entgegenhaltungen:
- EP-A- 0 632 010
- EP-A- 0 643 036
- DE-A- 1 668 730

## Beschreibung

Die Erfindung betrifft ein verbessertes Verfahren zur Herstellung von Diestern (oder neutralen Estern) von α,ω-Dicarbonsäuren aus cyclischen Ketonen und Kohlensäureestern unter Mitwirkung eines stark basischen Mittels.

Diester von höheren α, ω -Dicarbonsäuren, wie Brassylsäuredialkylester, sind wertvolle Zwischenprodukte, z.B. für die Herstellung von Riechstoffen, Wirkstoffen für Arznei- oder Pflanzenschutzmittel sowie von Oligo- oder Polyestern. Der cyclische Ester der Brassylsäure (die 13 Kohlenstoffatome hat) mit Ethylenglykol ist ein synthetischer moschusartiger Riechstoff, der in der Parfümerie und in der Kosmetik verwendet wird.

Die offengelegte deutsche Patentanmeldung 16 68 730 beschreibt die Herstellung von Brassylsäuredialkylestern durch Umsetzung von Cyclododecanon mit einem Dialkylcarbonat unter Verwendung eines stark basischen Mittels, das vorzugsweise in einem Verhältnis von etwa 1 Mol je Mol Cyclododecanon eingesetzt wird. Als geeignete stark basische Kondensationsmittel werden Kalium, Lithium sowie deren Alkoholate genannt. In den Beispielen werden Natrium, Natriummethylat. Natriumethylat und Lithium verwendet. Zur Aufarbeitung wird das Reaktionsgemisch mit Mineralsäure angesäuert, so daß als Nebenprodukt stöchiometrische Mengen an Salz entstehen. Das Verfahren arbeitet absatzweise und ergibt Ausbeuten an Brassylsäuredialkylestern von 85 bis 88 % der Theorie. Die Gesamtreaktion - Stufen (1) und (2) - der Synthese von Brassylsäuredimethylester (II) durch Umsetzung von Cyclododecanon mit Dimethylcarbonat unter Verwendung von Natriummethylat als stark basischem Mittel und Ansäuern des Reaktionsgemisches mit Salzsäure läßt sich sich durch das folgende Formelschema beschreiben:

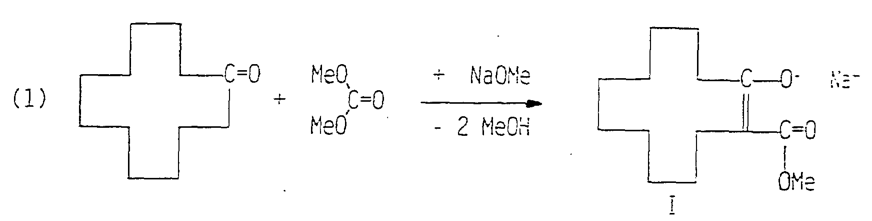

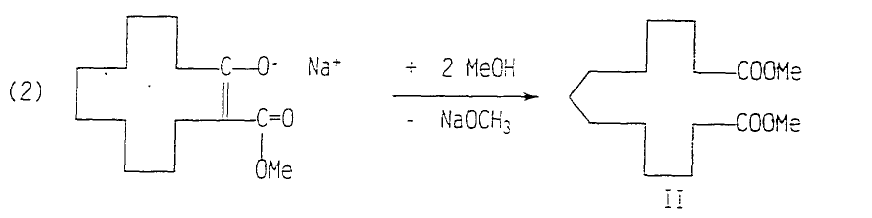

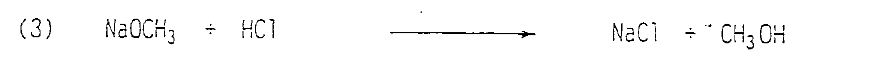

Die Teilreaktion (1), d.h. die Kondensation des Cyclododecanons zum β-Ketosäureester (I), im obigen Formelschema in Form seines Natriumenolats dargestellt, ist bereits von S.J.Rhoads et al. in Tetrahedron, 19 (1963). 1625 beschrieben worden. Als basisches Kondensationsmittel wurde dabei Natriumhydrid benutzt.

Nachteilig bei dem Verfahren nach der offengelegten deutschen Patentanmeldung ist allerdings, daß infolge der Verwendung von Natrium oder Natrium-haltigen basischen Kondensationsmitteln in molaren Mengen die rheologischen Eigenschaften des Reaktionsgemisches ungünstig sind. Es entstehen thixotrope Suspensionen, die schwer rührbar bis fast steif und daher namentlich im technischen Maßstab nur mit erheblichem Aufwand beherrschbar sind. Darüber hinaus entstehen beträchtliche Mengen an Nebenprodukten, wie Dialkylether, die dampfförmig entweichen, und das Natriumsalz des Kohlensäuremonoalkylesters, das in fester Form zurückbleibt. Beide Arten von Nebenprodukten führen bei der Synthese im technischen Maßstab zu Entsorgungsproblemen, ebenso die Natriumsalze, die bei der Neutralisation des Reaktionsgemisches mit Mineralsäure in molaren Mengen anfallen.

In EP-A-0 643 036 werden Pimelinsäureester aus Cyclohexanon hergestellt. Dabei werden mit vorzugsweise 1 bis 3 Äquivalenten große Mengen an Alkoholat eingesetzt. Dadurch fallen als Nebenprodukt Salze in erheblichen Mengen an.

EP-A-0 632 010 beschreibt die Herstellung auch langkettiger Dicarbonsäuren aus großen cyclischen Ketonen. Dabei wird ein Mineralsalzanfall durch Anwendung von kleinen Mengen stickstoffhaltiger Basen, vorzugsweise von Aminen, vermieden. Die Amine führen aber, wenn die langkettigen Ester zu Riechstoffen verarbeitet werden, auch im ppm-Bereich zu unerwünschten Nebengerüchen. Außerdem wird bei der Entsorgung durch die Anwendung von Aminen der Stickstoffgehalt der Abwässer erhöht.

Es wurde nun gefunden, daß sich Diester höher α, ω-Dicarbonsäuren vorteilhaft herstellen lassen, wenn man ein cycloaliphatisches Keton mit 8 bis 20 Ringgliedern und α-ständiger Methylengruppe, mindestens äquimolare Mengen eines Kohlensäurediesters und 0,05 bis 0,5 Äquivalente eines Kaliumalkanolats pro Mol cycloaliphatisches Keton kontinuierlich einer Reaktionszone zuführt, in der die Kondensation des cycloaliphatischen Ketons mit dem Kohlensäurediester zum entsprechenden cyclischen β-Ketosäureesterenolat und dessen Ringspaltung zum α, ω-Dicarbonsäurediester durch frei gewordenen Alkohol stattfinden und aus der man kontinuierlich Reaktionsgemisch abzieht.

Besonders vorteilhaft führt man das Verfahren nach der Erfindung aus, indem man die Reaktionszone in mindestens zwei Teilreaktionszonen unterteilt, in zumindest einer Teilreaktionszone leichtsiedende Anteile aus dem Reaktionsgemisch entfernt und in zumindest einer weiteren, späteren Teilreaktionszone dem Reaktionsgemisch Alkohol zuführt.

Das Verfahren nach der Erfindung ergibt höhere Ausbeuten, nämlich mehr als 90 % der Theorie, als die. Verfahren nach dem Stand der Technik. Da nur katalytische Mengen an stark basischem Stoff benötigt werden, sind die erwähnten rheologischen Probleme weniger ausgeprägt und auch im technischen Maßstab ohne besondere Maßnahmen beherrschbar. Überraschenderweise sind die rheologischen Eigenschaften der Reaktionsgemische gegenüber einer Arbeitsweise mit den entsprechenden Mengen an Natrium bzw. Natriumverbindungen unter sonst gleichen Bedingungen erheblich verbessert. Die nur katalytischen Mengen an Kaliumalkanolat bedeuten natürlich auch einen Kostenvorteil gegenüber der Arbeitsweise mit molaren Mengen und führen außerdem zu geringerer Bildung von Nebenprodukten, wie Dialkylethern und Salzen des Kohlensäuremonoesters, sowie an Salzen bei der Neutralisierung des Kaliumalkanolats. Weiterhin ist die Energiebilanz des Verfahrens nach der Erfindung deutlich günstiger als die der bekannten diskontinuierlichen Arbeitsweise. Beim Mischen des cycloaliphatischen Ketons mit dem Kohlensäurediester und dem Kaliumalkanolat wird eine beträchtliche Wärmemenge frei. Sie beträgt im Falle von molaren Mengen an Cyclododecanon. Dimethylcarbonat und Natriummethylat -30 kJ pro Mol Cyclododecanon. Beim bekannten diskontinuierlichen Verfahren muß man daher anfangs kühlen und zum Ende der Reaktion heizen. Die dadurch erforderliche Umstellung von Kühlung auf Heizung ist regelungs- und sicherheitstechnisch anspruchsvoll und vernichtet zudem viel Energie. Das kontinuierliche Verfahren nach der Erfindung arbeitet dagegen nahezu adiabatisch und isotherm. Im stationären Zustand muß lediglich eine kleine, konstante Wärmemenge zugeführt werden.

Das Verfahren geht von cycloaliphatischen Ketonen mit 8 bis 20 Kohlenstoffatomen und α-ständiger Methylengruppe aus und führt zu Diestern von α,ω-Dicarbonsäuren, die ein Kohlenstoffatom mehr aufweisen als das Ausgangsketon. also 9 bis 21 Kohlenstoffatome. Bevorzugte Ausgangsketone sind Cycloalkanone mit 8 bis 20. insbesondere mit 10 bis 16 Kohlenstoffatomen, wie Cyclooctanon, Cyclodecanon, Cyclododecanon, Cyclopentadecanon, Cyclahexadecanon, Cyclooctadecanon und Cycloeicosanon.

Als Kohlensäurediester eignen sich für das Verfahren nach der Erfindung insbesondere die Ester mit zwei in der Regel gleichen, über Sauerstoff an den Carbonylkohlenstoff gebundenen Kohlenwasserstoffresten, inbesondere Alkyl-, Cycloalkyl-, Aralkyl-, Alkaryl- oder Arylresten. Besonders bevorzugt werden Dialkylcarbonate mit gleichen Alkylresten mit jeweils 1 bis 6 Kohlenstoffatomen. Die Kohlensäurediester liefern die eine der beiden Carbonestergruppen des herzustellenden α.ω-Dicarbonsäurediesters. Von den geeigneten Kohlensäurediestern seien z.B. Dimethylcarbonat. Diethylcarbonat. Diisopropylcarbonat. Di-n-butylcarbonat. Dicyclopentylcarbonat. Dibenzylcarbonat. Diphenylcarbonat und Di-p-tolylcarbonat genannt. Der Kohlensäurediester sollte in mindestens äquimolaren Mengen, bezogen auf das cycloaliphatische Keton, verwendet werden. Gegebenenfalls wirkt ein Überschuß, beispielsweise bis zur 2.5-fachen molaren Menge. als Lösungs- und Verdünnungsmittel.

Geeignete Kaliumalkanolate sind z. B. Kaliummethylat. Kaliumethylat, Kaliumisopropylat, Kalium-n-butylat und Kaliumbenzylat. Das Alkanolat sollte sich zweckmäßig von dem Alkohol ableiten, dessen Kohlensäurediester die eine der beiden Carbonestergruppen des herzustellenden α,ω-Dicarbonsäurediesters liefert. Anderenfalls muß man mit der Entstehung von gemischten Estern der betreffenden α, ω-Dicarbonsäure rechnen.

Vorzugsweise verwendet man das Kaliumalkanolat in Mengen von 0,1 bis 0,3 Äquivalenten pro Mol cycloaliphatisches Keton.

Das Verfahren nach der Erfindung kann in Gegenwart inerter Lösungs-und Verdünnungsmittel durchgeführt werden. Dies empfiehlt sich besonders dann, wenn ein nur geringer Überschuß an Kohlensäurediester eingesetzt wird. Inerte Lösungs- und Verdünnungsmittel erleichtern auch die Zuführung von Alkoholaten oder anderen pulverförmigen stark basischen Mitteln, weil Suspensionen leichter dosierbar sind als Pulver. Geeignete inerte Lösungs- und Verdünnungsmittel sind z.B. Glykolether, wie Ethylenglykoldiethylether und Triethylenglykoldimethylether, sowie N-substituierte Carbonsäureamide. wie Dimethylformamid und N-Methylpyrrolidon, oder das als Reaktionspartner eingesetzte Dialkylcarbonat. welches mit den Alkoholaten keine Reaktion eingeht. Im allgemeinen werden inerte Lösungs- und Verdünnungsmittel, falls überhaupt, in Mengen von etwa 5 bis etwa 50 Gewichtsprozent, bezogen auf das gesamte Reaktionsgemisch, eingesetzt.

Wenn man das Verfahren nach der Erfindung in nur einer Reaktionszone durchführt, laufen die den obigen Teilreaktionen (1) und (2) entsprechenden Reaktionen nebeneinander ab. Man führt das cycloaliphatische Keton, den Kohlensäurediester, das stark basische Mittel, gegebenenfalls als Suspension in einem inerten Lösungs- und Verdünnungsmittel, in den angegebenen Mengen kontinuierlich in die Reaktionszone ein und zieht kontinuierlich die entsprechende Menge Reaktionsgemisch ab. Die Temperaturen in der Reaktionszone hängen von den jeweiligen Ausgangsstoffen und der angestrebten Verweilzeit ab. Sie betragen im allgemeinen 40 bis 160°C, insbesondere 60 bis 120°C und werden, wie erwähnt, im stationären Zustand durch geringe Wärmezufuhr aufrechterhalten. Die mittleren Verweilzeiten betragen, je nach Ausgangsstoffen und Reaktionstemperatur, im allgemeinen 2 bis 10 Stunden.

Besonders günstige Ergebnisse werden erzielt, wenn man die Reaktionszone in mindestens zwei Teilreaktionszonen unterteilt, in mindestens einer ersten Teilreaktionszone leichtsiedende Anteile aus dem Reaktionsgemisch entfernt und in mindestens einer zweiten, späteren (also im Verfahrensgang nachgeschalteten) Teilreaktionszone dem Reaktionsgemisch Alkohol zuführt. Auf diese Weise wird die obige Gesamtreaktion (1) und (2) so unterteilt, daß die Teilreaktion (1) im wesentlichen in einer ersten Teilreaktionszone (in der Leichtsieder abgenommen werden) und die Reaktion (2) im wesentlichen in einer zweiten, späteren Teilreaktionszone (in der Alkohol zugeführt wird) abläuft. Wenn also die Reaktionszone in drei Teilreaktionszonen unterteilt ist und in der ersten Teilreaktionszone keine leicht siedenden Anteile abgezogen werden, dann muß dies in der zweiten Teilreaktionszone geschehen, und in der dritten Teilreaktionszone muß dem Reaktionsgemisch Alkohol zugeführt werden. Bei den leichtsiedenden Anteilen handelt es sich überwiegend um Kohlensäurediester, in der Teilreaktion (1) frei werdenden Alkohol sowie um Ether und andere Nebenprodukte, die in geringerem Maße auch bei dem Verfahren nach der Erfindung entstehen. Diese leichtsiedenden Anteile können kontinuierlich oder absatzweise durch Destillation aufgearbeitet werden. worauf der Kohlensäurediester in die erste Teilreaktionszone zurückgeführt und der Alkohol einer zweiten und/oder auch etwaigen weiteren Teilreaktionszonen zugeführt werden kann. Natürlich ist es auch möglich, dem Reaktionsgemisch in der zweiten und/oder etwaigen weiteren Teilreaktionszonen frischen Alkohol zuzuführen. Auf jeden Fall ist es zweckmäßig, denjenigen Alkohol zuzuführen, der dem eingesetzten Kohlensäurediester entspricht. Anderenfalls sind die Reaktionsprodukte gemischte Diester der betreffenden α,ω-Dicarbonsäure. Diese sind zwar für manche Zwecke ebenso oder ähnlich brauchbar wie die Diester mit gleichen Alkoholresten. Im allgemeinen werden aber die letzteren bevorzugt.

Hinsichtlich der Reaktionstemperaturen unterscheidet sich die Arbeitsweise mit Teilreaktionszonen nicht wesentlich von dem Verfahren mit nur einer Reaktionszone. Sie betragen also im allgemeinen 40 bis 160 °C, vorteilhaft 60 bis 120°C. Dabei kann es zweckmäßig sein, die Temperatur in der oder den Teilreaktionszonen, in denen Alkohol zugeführt wird, etwas höher einzustellen als in der oder den Teilrektionszonen, in denen leichtsiedende Anteile aus dem Reaktionsgemisch entfernt werden. Wie bei der Ausführungsform des Verfahrens nach der Erfindung mit nur einer Reaktionszone, muß man auch bei der Variante mit unterteilter Reaktionszone im stationären Zustand in geringem Maße Energie zuführen, und zwar der oder den späteren Teilreaktionszonen mehr als der oder den früheren Teilreaktionszonen. Die optimalen Temperaturen in den einzelnen Teilreaktionszonen hängen u.a. von den jeweiligen Reaktionsteilnehmern ab und lassen sich durch orientierende Versuche unschwer ermitteln. Im allgemeinen herrscht in den Teilreaktionszonen (wie auch bei der Ausführungsform des Verfahrens mit nur einer Reaktionszone) Atmosphärendruck. Wenn man im Interesse einer hohen Reaktionsgeschwindigkeit Temperaturen am oberen Ende der genannten Bereiche wählt und Ester niedrig siedender Alkohole hergestellt werden sollen, kann man auch erhöhte Drücke, z.B. von 2 bis 10 bar, anwenden. Die mittleren Verweilzeiten in den einzelnen Teilreaktionszonen können in weiten Grenzen schwanken und betragen im allgemeinen zwischen 30 Minuten und 20 Stunden. Dabei kann es zweckmäßig sein, für die erste oder die frühen Teilreaktionszonen kürzere Verweilzeiten vorzusehen als für die letzte oder die späteren Teilreaktionszonen.

Das Verfahren nach der Erfindung mit nur einer Reaktionszone kann z.B. in einem Rührkessel durchgeführt werden, dem man die Ausgangsstoffe einschließlich des Kaliumalkanolats und gegebenenfalls eines inerten Lösungs- und Verdünnungsmittels im gewünschten Mengenverhältnis kontinuierlich zuführt. Die Reaktionszone ist dann der Innenraum des Rührkessels. Im allgemeinen kann man die Reaktionsparameter. wie Stoffmengen, Reaktionstemperatur und mittlere Verweilzeit. so aufeinander abstimmen, daß die Reaktion im stationären Zustand isotherm und zugleich praktisch adiabatisch verläuft. Ein Reaktor mit Vorrichtungen zum Heizen und Kühlen ist jedoch vorteilhaft, weil er die erwünschte Flexibilität der Reaktionsführung bietet.

Das Verfahren nach der Erfindung mit zwei oder mehr Teilreaktionszonen kann z.B. in einer Kaskade aus zwei oder mehr hintereinandergeschalteten Rührreaktoren durchgeführt werden. Dabei werden dem ersten Reaktor kontinuierlich das cycloaliphatische Keton, der Kohlensäurediester, das Kaliumalkanolat und gegebenenfalls ein inertes Lösungs und Verdünnungsmittel in den angegebenen Mengenverhältnissen zugeführt. Aus dem ersten oder einem der folgenden Reaktoren der Kaskade destillieren leicht siedende Anteile ab und werden kondensiert. Die Menge der abdestillierenden Leichtsieder ergibt sich zwangsläufig aus der Art und Menge der Ausgangsstoffe sowie der Temperatur und dem Druck, die in der ersten Teilreaktionszone herrschen. Sie beträgt im allgemeinen 50 bis 250 g je 1 Reaktionsgemisch und Stunde. Die Leichtsieder werden absatzweise oder kontinuierlich durch Destillation aufgearbeitet. Der Kohlensäurediester wird in den ersten Reaktor zurückgeführt, zweckmäßig zusammen mit den Ausgangsstoffen. Der Alkohol wird dem nächsten oder einem folgenden Reaktor zugeführt.

Sobald im ersten Reaktor die angestrebte Füllhöhe erreicht ist, wird ihm eine Menge an Reaktionsgemisch entnommen, die den zugeführten Ausgangsstoffen, gegebenenfalls abzüglich der abdestillierten Leichtsieder, entspricht, und in einen zweiten Reaktor überführt. Aus diesem können dann Leichtsieder abdestilliert werden, wenn dies nicht schon im ersten Reaktor geschehen ist. Anderenfalls wird diesem Reaktor sowie gegebenenfalls weiteren Reaktoren der Kaskade Alkohol zugeführt, vorteilhaft 10 bis 50 Gewichtsteile pro Gewichtsteil eingesetztes cycloaliphatisches Keton. Wenn mehreren Reaktoren der Kaskade Alkohol zugeführt wird, so kann dessen Gesamtmenge im Verhältnis der Volumina der Reaktoren und damit der mittleren Verweilzeiten aufgeteilt werden.

Das Reaktionsgemisch, das - bei der Ausführungsform des Verfahrens mit nur einer Reaktionszone - den einzigen Reaktor oder - bei Ausführungsformen mit mehreren Teilreaktionszonen - den letzten Reaktor der Kaskade verläßt, ist im allgemeinen bei Temperaturen um 50°C hinreichend dünnflüssig, so daß es durch mechanische Pumpen gut gefördert werden kann. Gegebenenfalls kann man inerte Lösungs- oder Verdünnungsmittel, wie Toluol. Xylol oder Diethylenglykoldimethylether, zusetzen, um das Gemisch pumpfähig zu erhalten. Dies empfiehlt sich besonders dann, wenn der hergestellte α,ω-Dicarbonsäurediester, der in einer konzentrierten Lösung anfällt, einen relativ hohen Schmelzpunkt hat. Brassylsäuredimethylester z.B. schmilzt bei 33-35°C. Aus dem Reaktionsgemisch kann man, z.B. mittels eines üblichen Dünnschichtverdampfers. kontinuierlich oder absatzweise leicht flüchtige Anteile abtrennen. Diese bestehen überwiegend aus Alkohol sowie Kohlensäurediester und werden in das Verfahren zurückgeführt. Den Rückstand aus dem Dünnschichtverdampfer kann man in einem zweiten Trennschritt, z.B. einer Kurzwegdestillation, in den gewünschten α,ω-Dicarbonsäurediester und einen Rückstand trennen, der noch höher siedende oder nicht mehr destillierbare Anteile enthält. Der Ester kann gewünschtenfalls durch fraktionierte Destillation weiter gereinigt werden. Der Rückstand enthält das Kaliumalkanolat und kann in den einzigen Reaktor bzw. den ersten Reaktor der Kaskade zurückgeführt werden. Zweckmäßig schleust man allerdings einen Teil des Rückstands aus, um unerwünschte und gegebenenfalls störende hochsiedende oder nicht destillierbare Nebenprodukte aus dem Kreislauf zu entfernen.

Alternativ kann man das Reaktionsgemisch, das den einzigen Reaktor bzw. den letzten Reaktor der Kaskade verläßt, mit Wasser behandeln, wobei sich zwei gut voneinander trennbare Phasen ausbilden. Die untere, wäßrige Phase enthält verhältnismäßig geringe Mengen Salz. weil das Kaliumalkanolat nur in katalytischen Mengen eingesetzt wurde. Sie kann daher ohne großen Aufwand entsorgt oder umweltverträglich aufgearbeitet werden. Die obere, organische Phase kann ohne hohen Trennaufwand durch Destillation gereinigt werden.

Die folgenden Beispiele sollen die Erfindung weiter erläutern, nicht aber ihren Umfang begrenzen.

### Beispiel 1

In einen 1 l-Doppelwandglasreaktor (erster Reaktor) mit Temperaturregelung. Rührer und Destillationsaufsatz werden stündlich 182 g (1 mol) Cyclododecanon. 162 g (1,8 mol) Dimethylcarbonat sowie eine Suspension von 14 g (0,2 mol) Kaliummethylat-Pulver in 100 g Triethylenglykoldimethylether eindosiert. Die Stoffe werden durch Rühren gemischt, und die Temperatur wird zunächst durch Kühlen auf 90°C gehalten. Nach einer Stunde ist der vorgesehene Füllstand im Reaktor erreicht und man beginnt mit dem Abdestillieren von Leichtsiedern und der Abnahme von Reaktionsgemisch. Die mittlere Verweilzeit im ersten Reaktor beträgt also 1 Stunde. Es werden stündlich ca. 50 g Leichtsieder abgenommen, die zu ca. 25% aus Dimethylcarbonat und zu ca. 75% aus Methanol bestehen. Die Leichtsieder können zur Rückgewinnung dieser Bestandteile aufgearbeitet werden. Nach etwa 4 Stunden herrscht im ersten Reaktor stationärer Zustand, und es bedarf dann einer geringen Wärmezufuhr, um die Temperatur von 90°C zu halten.

Dem ersten Reaktor wird soviel Reaktionsprodukt entnommen. daß der Füllstand erhalten bleibt, und mittels einer Dosierpumpe in einen zweiten, 5 1 fassenden Glasreaktor (zweiter Reaktor) gefördert. Nach 8 Stunden ist der vorgesehene Füllstand im zweiten Reaktor erreicht. und man beginnt mit der Entnahme von ca. 460 g/h Reaktionsprodukt zur Aufarbeitung, so daß der Füllstand gehalten wird. Die mittlere Verweilzeit im zweiten Reaktor beträgt also etwa 8 Stunden. Das Reaktionsgemisch wird auf 90-100°C gehalten, und es werden stündlich 50 g Methanol in den zweiten Reaktor eingebracht.

Nach ca. 2 Stunden, gerechnet vom Beginn der Zuführung von Reaktionsgemisch aus dem ersten Reaktor, herrscht auch im zweiten Reaktor stationärer Zustand. Die ca. 460 g/h Reaktionsgemisch werden mittels eines Labordünnschichtverdampfers in ein Leichtsiedergemisch, im wesentlichen bestehend aus Methanol und Dimethylcarbonat. und einen Sumpfaustrag zerlegt. Dieser wird in einem zweiten Destillationsschritt über eine Kurzwegdestillation bei 1 hPa in eine destillierbare Fraktion (Kopftemperatur von 95 auf 135°C ansteigend) und einen nicht destillierbaren Rückstand getrennt. Aus der destillierbaren Fraktion werden in einer anschließenden fraktionierten Destillation neben dem Triethylenglykoldimethylether 252 g Brassylsäuredimethylester vom Kp. 112°C/0,5 hPa, Fp. 34-35°C mit einer Reinheit (nach GC-Analyse) von 99 % gewonnen. Die Ausbeute, bezogen auf eingesetztes Cyclododecanon, beträgt 92 % der Theorie.

### Beispiel 2

In einen 2 1-Doppelwandglasreaktor mit Pulverdosiervorrichtung (erster Reaktor) werden innerhalb einer Stunde 14 g (0,2 mol) Kaliummethylat-Pulver zu einem Gemisch aus 182 g (1 mol) Cyclododecanon und 144 g (1,6 mol) Dimethylcarbonat dosiert. Dabei wird die Temperatur durch Kühlung auf 60°C gehalten. Nachdem diese Grundmischung hergestellt ist, werden stündlich 56 g (0,8 mol) Kaliummethylat, 728 g (4 mol) Cyclododecanon und 576 g (6,4 mol) Dimethylcarbonat zudosiert. Nach etwa 2 Stunden ist stationärer Zustand erreicht, in dem der Reaktor durch geringe Wärmezufuhr auf einer Temperatur von 60°C gehalten wird. Dem ersten Reaktor werden stündlich ca. 1.388 g Reaktionsgemisch entnommen, so daß der Füllstand gehalten wird. Die mittlere Verweilzeit beträgt also etwa 1 Stunde.

Der Austrag aus dem ersten Reaktor wird in einen 10 1-Reaktor mit Destillationsaufsatz (zweiter Reaktor) überführt. Die Temperatur des Gemisches wird auf 100°C gehalten, und über den Destillationsaufsatz werden stündlich ca. 200 g Leichtsieder abgezogen, die Methanol und Dimethylcarbonat enthalten. Nach 8 Stunden ist der vorgesehene Füllstand im zweiten Reaktor erreicht. Man beginnt mit der Entnahme von 1.200 g/h Reaktionsgemisch, so daß der Füllstand gehalten wird. Die mittlere Verweilzeit im zweiten Reaktor beträgt also etwa 8 Stunden. Nach etwa 10 Stunden, gerechnet vom Beginn der Zuführung von Reaktionsgemisch in den zweiten Reaktor, hat sich ein stationärer Zustand eingestellt.

Das dem zweiten Reaktor entnommene Reaktionsgemisch wird in einen 51-Reaktor (dritter Reaktor) überführt, in dem durch Wärmezufuhr eine Temperatur von 70 °C gehalten wird. Dem dritten Reaktor werden weiterhin 100 g/h Methanol zugeführt. Nach etwa 2 Stunden ist der vorgesehen Füllstand erreicht, und nach weiteren 2 Stunden hat sich ein stationärer Zustand eingestellt. Die mittlere Verweilzeit beträgt etwa 2 Stunden. Aus dem dritten Reaktor werden stündlich 1.300 g Reaktionsgemisch abgezogen, die wie in Beispiel 1 aufgearbeitet werden. Man erhält Brassylsäuredimethylester mit einer Reinheit (nach GC) von 98,1 %. Die Ausbeute, bezogen auf die stündlich zugeführten 4 mol Cyclododecanon, beträgt 91 der Theorie.

### Beispiel 3

Man verfährt wie in Beispiel 2, führt jedoch dem ersten Reaktor stündlich 100 g (1,2 mol) Kaliummethylat, 728 g (4 mol) Cyclododecanon und 849 g (7,2 mol) Diethylcarbonat zu. Statt 100 g/h Methanol werden dem Reaktor 120 g/h Ethanol zugeführt. Man erhält stündlich 1.116 g Brassylsäurediethylester entsprechend einer Ausbeute von 93 % der Theorie, bezogen auf eingesetztes Cyclododecanon.

### Beispiel 4

Man verfährt wie in Beispiel 1, führt jedoch dem ersten Reaktor an Stelle von Kaliummethylat-Pulver mittels einer Dosierpumpe eine Suspension von 14 g (0,2 mol) Kaliummethylat-Pulver in 80 g ( 0,9 mol) Dimethylcarbonat zu. Gleichzeitig wird dem ersten Reaktor ein Gemisch aus 100 g (1,1 mol) Dimethylcarbonat und 182 g ( 1 mol) Cyclododecanon zugeführt. Man erhält stündlich 250 g Brassylsäuredimethylester, entsprechend einer Ausbeute von 92 % der Theorie, bezogen auf eingesetztes Cyclododecanon.

## Patentansprüche

1. Verfahren zur Herstellung von Diestern höherer α,ω-Dicarbonsäuren aus cycloaliphatischen Ketonen und Kohlensäurediestern in Gegenwart eines stark basischen Mittels,
dadurch gekennzeichnet,
daß man ein cycloaliphatisches Keton mit 8 bis 20 Ringgliedern und α-ständiger Methylengruppe, mindestens äquimolare Mengen eines Kohlensäurediesters und 0,05 bis 0,5 Äquivalente pro Mol cycloaliphatisches Keton eines Kaliumalkanolats kontinuierlich einer Reaktionszone zuführt, in der die Kondensation des cycloaliphatischen Ketons mit dem Kohlensäurediester zum entsprechenden cyclischen β-Ketosäureesterenolat und dessen Ringspaltung zum α,ω-Dicarbonsäurediester durch frei gewordenen Alkohol stattfinden und aus der man kontinuierlich Reaktionsgemisch abzieht.

2. Verfahren nach Anspruch 1,
dadurch gekennzeichnet,
daß man die Reaktionszone in mindestens zwei Teilreaktionszonen unterteilt, in zumindest einer der Teilreaktionszonen leichtsiedende Anteile aus dem Reaktionsgemisch entfernt und in zumindest einer weiteren, späteren Teilreaktionszone dem Reaktionsgemisch Alkohol zuführt.

3. Verfahren nach einem der Ansprüche 1 oder 2,
dadurch gekennzeichnet,
daß man Kaliummethylat verwendet.

4. Verfahren nach einem der Ansprüche 1 bis 3,
dadurch gekennzeichnet,
daß man Cyclododecanon als cycloaliphatisches Keton verwendet.

5. Verfahren nach einem der Ansprüche 1 bis 4,
dadurch gekennzeichnet,
daß man als Kohlensäurediester einen Ester der Kohlensäure mit einem Alkanol mit 1 bis 6 Kohlenstoffatomen verwendet.

6. Verfahren nach einem der Ansprüche 1 bis 4,
dadurch gekennzeichnet,
daß man als Kohlensäurediester Dimethylcarbonat verwendet.

7. Verfahren nach einem der Ansprüche 1 bis 6,
dadurch gekennzeichnet,
daß die Temperatur in der Reaktionszone bzw. in den Teilreaktionszonen 40 bis 160 °C beträgt.

8. Verfahren nach einem der Ansprüche 1 bis 6,
dadurch gekennzeichnet,
daß die Temperatur in der Reaktionszone bzw. in den Teilreaktionszonen 60 bis 120 °C beträgt.

9. Verfahren nach einem der Ansprüche 1 bis 8,
dadurch gekennzeichnet,
daß man das Kaliumalkanolat als Suspension in einem inerten Lösungs- und Verdünnungsmittel in die Reaktionszone bzw. in die erste Teilreaktionszone einführt.

10. Verfahren nach einem der Ansprüche 1 bis 9,
dadurch gekennzeichnet,
daß man dem Reaktionsgemisch vor der Aufarbeitung zur Verbesserung der Pumpfähigkeit ein inertes Lösungs- und Verdünnungsmittel zusetzt.

## Claims

1. A process for preparing a diester of a higher α,ω-dicarboxylic acid from a cycloaliphatic ketone and a carbonic diester in the presence of a strongly basic agent, characterized in that a cycloaliphatic ketone having 8 to 20 ring members and an α-methylene group, at least an equimolar amount of a carbonic diester and from 0.05 to 0.5 equivalent per mole of cycloaliphatic ketone of a potassium alkanolate are continuously fed to a reaction zone in which condensation of the cycloaliphatic ketone with the carbonic diester to give the corresponding cyclic β-keto acid ester enolate and the ring-opening of the latter by the released alcohol to give the α,ω-dicarboxylic diester take place and from which reaction mixture is continuously taken off.

2. A process according to claim 1, characterized in that the reaction zone is subdivided into at least two part-reaction zones, in at least one of the part-reaction zones low-boiling portions are removed from the reaction mixture, and in at least one further, later part-reaction zone alcohol is fed to the reaction mixture.

3. A process according to either one of claims 1 and 2, characterized in that potassium methylate is used.

4. A process according any one of claims 1 to 3, characterized in that cyclododecanone is used as cycloaliphatic ketone.

5. A process according to any one of claims 1 to 4, characterized in that an ester of carbonic acid with an alkanol having 1 to 6 carbon atoms is used as carbonic diester.

6. A process according to any one of claims 1 to 4, characterized in that dimethyl carbonate is used as carbonic diester.

7. A process according to any one of claims 1 to 6, characterized in that the temperature in the reaction zone or in the part-reaction zones is 40 to 160°C.

8. A process according to any one of claims 1 to 6, characterized in that the temperature in the reaction zone or in the part-reaction zones is 60 to 120°C.

9. A process according to any one of claims 1 to 8, characterized in that the potassium alkanolate is introduced into the reaction zone or into the first part-reaction zone as a suspension in an inert solvent and diluent.

10. A process according to any one of claims 1 to 9, characterized in that an inert solvent and diluent is added to the reaction mixture before work-up to improve the pumpability.

## Revendications

1. Procédé de préparation de diesters d'acides α,ω-dicarboxyliques supérieurs, à partir de cétones cycloaliphatiques et de diesters d'acide carbonique en présence d'un agent fortement basique,
caractérisé en ce qu'
on amène en continu une cétone cycloaliphatique ayant de 8 à 20 éléments de cycle et un groupe méthylène en position α, des quantités au moins équimolaires d'un diester d'acide carbonique et de 0,05 à 0,5 équivalents par mole de cétone cycloaliphatique d'un alkanolate de potassium, à une zone de réaction dans laquelle la condensation de la cétone cycloaliphatique avec le diester d'acide carbonique en l'énolate d'ester d'acide β-cétonique cyclique correspondant et sa coupure de cycle en diester d'acide α, ω-dicarboxylique se produisent grâce à l'alcool libéré, et à partir de laquelle on soutire en continu le mélange réactionnel,

2. Procédé selon la revendication 1,
caractérisé en ce qu'
on subdivise la zone de réaction en au moins deux zones de réaction partielles, on élimine dans au moins une des zones de réactions partielles les fractions à bas point d'ébullition du mélange réactionnel et on amène dans au moins une autre zone de réaction partielle ultérieure l'alcool au mélange réactionnel.

3. Procédé selon l'une des revendications 1 ou 2,
caractérisé en ce qu'
on utilise du méthylate de potassium.

4. Procédé selon l'une quelconque des revendications 1 à 3,
caractérisé en ce qu'
on utilise la cyclododécanone en tant que cétone cycloaliphatique.

5. Procédé selon l'une quelconque des revendications 1 à 4,
caractérisé en ce qu'
on utilise en tant que diester d'acide carbonique un ester d'acide carbonique avec un alkanol ayant de 1 à 6 atomes de carbone.

6. Procédé selon l'une quelconque des revendications 1 à 4,
caractérisé en ce qu'
on utilise en tant que diester d'acide carbonique du carbonate de diméthyle.

7. Procédé selon l'une quelconque des revendications 1 à 6,
caractérisé en ce que
la température dans la zone de réaction ou dans les zones de réaction partielles s'élève de 40 à 160°C.

8. Procédé selon l'une quelconque des revendications 1 à 6,
caractérisé en ce que
la température dans la zone de réaction ou dans les zones de réaction partielles s'élève de 60 à 120°C.

9. Procédé selon l'une quelconque des revendications 1 à 8,
caractérisé en ce qu'
on introduit l'alkanolate de potassium sous forme de suspension dans un solvant de dilution inerte, dans la zone de réaction ou dans la première zone de réaction partielle.

10. Procédé selon l'une quelconque des revendications 1 à 9,
caractérisé en ce qu'
on ajoute au mélange réactionnel avant le traitement, en vue de l'amélioration de l'aptitude au pompage un solvant de dilution inerte.
